# EUROPEAN PATENT APPLICATION

(11) **EP 1 426 762 A1**
(43) Date of publication of application: **09.06.2004**
(21) Application number: 02760568.2
(22) Date of filing: 07.08.2002
(51) Int. Cl.: G01N 33/15, G01N 33/50

(54) **REMEDIES FOR HEART DISEASES**

(30) Priority: 08.08.2001 JP 2001240852; 19.11.2001 JP 2001353047; 28.03.2002 JP 2002092363
(71) Applicant: CHUGAI SEIYAKU KABUSHIKI KAISHA, Tokyo, 115-8543 (JP)
(72) Inventor: OKADA, Yasunobu, Okazaki-shi, Aichi 444-0877 (JP); TANABE, Shigeru, c/o Chugai Seiyaku K.K., Gotenba-shi, Shizuoka 412-8513 (JP)
(74) Representative: VOSSIUS & PARTNER
(86) International application number: PCT/JP2002/008069
(87) International publication number: WO 2003/014727

(57) **Abstract**

This invention aims at providing a method of searching for substances that can selectively suppress apoptosis in cardiovascular cells.

This objective is attained by providing a method of screening for therapeutic and/or prophylactic agents of cardiac disease, which comprises the steps of inducing apoptosis in cultured myocardial cells and/or cultured vascular endothelial cells; treating the cells with a Cl⁻ channel blocker under test; and evaluating the therapeutic and/or prophylactic effect of the blocker under test on cardiac disease by checking to see if it can suppress apoptotic cell death in the cardiovascular cells or vascular endothelial cells.

## Description

### TECHNICAL FIELD

This invention relates to therapeutic or prophylactic agents of cardiac disease. More specifically, the invention relates to therapeutic or prophylactic agents of cardiac disease containing the volume-sensitive outwardly rectifying Cl⁻ channel blockers.

The invention also relates to a method of screening for such therapeutic or prophylactic agents of cardiac disease. More specifically, the invention relates to a method of screening for suitable therapeutic or prophylactic agents of cardiac disease by checking to see if apotosis induced by a known method can be inhibited by a Cl⁻ channel blocker under test.

### BACKGROUND ART

Most cases of cardiac disease are primarily due to structural or functional damage to the coronary artery which causes imbalance between blood supply from the coronary artery and its demand by the myocardium, eventually leading to acute or chronic ischemic myocardial dysfunction. If myocardial cells become ischemic, it is believed apoptosis consequently occurs in them. Once apoptosis has been triggered off, myocardial cells are incapable of avoiding apoptosis and cell death occurs, leading to serious conditions of symptomatic cardiac disease such as angina pectoris, myocardial infarction and heart failure.

In the process of apoptosis which is known as physiological cell death, it is known that progressive cell shrinkage first occurs, then followed by cell fragmentation (formation of apoptotic bodies). At the very early stage in the course of this apoptotic cell death which is prior to the formation of apoptotic bodies, there occurs normotonic cell shrinkage called apoptotic volume decrease (AVD) (Maeno, E. et al., Proc. Natl. Acad. Sci. U.S.A., Vol. 97, 9487-9492, 2000).

Induction of AVD under normotonic conditions is associated with facilitation of regulatory volume decrease (RVD) which occurs after cell swelling has been physicochemically compelled by extracellular hypotonic stresses. Both the AVD induction and the RVD facilitation are known to occur at the very early stage of apoptosis, preceding cytochrome c release from mitochondria, caspase (e.g. caspase-3) activation, DNA ladder formation and ultrastructural alterations.

AVD and RVD have been closely studied in many cell types including epithelial cell lines (e.g. human derived epithelial HeLa cell), lymphoid cell lines (e.g. human derived lymphoid U937 cell) and neuronal cell lines (e.g. rat pheochromocytoma PC12 cell and mouse neuroblastoma x rat glioma hybrid NG108-15 cell). As a result, it has been found that the aforementioned RVD is primarily caused by KCl efflux owing to parallel activation of the Ca²⁺-dependent K⁺ channel and the volume-sensitive outwardly rectifying Cl⁻ channel (VSOR-ClC, also called the volume regulated anion channel (VRAC) or the volume-sensitive organic osmolyte and anion channel (VSOAC)). Also it is known that if the AVD induction and RVD facilitation are inhibited by blocking the volume-regulatory Cl⁻ channel or K⁺ channel, the above-mentioned cells do not undergo any biochemical or morphological changes that accompany apoptosis and apoptotic cell death itself can be prevented.

It is known in many cells that the K⁺ channel is always activated if the cell is at quiescence state whereas the Cl⁻ channel is not activated unless it is necessary, except in limited cell types including skeletal muscle and erythrocyte. Therefore, VSOR-ClC is considered to play a more important role in the AVD induction and the RVD facilitation but its entity is yet to be unraveled.

Many studies have shown that different kinds of tissues as cell sources express different volume-regulatory Cl⁻ channels. For example, it has been suggested that ClC-3 is the entity of VSOR-ClC in myocardial cells (Duan et al., Nature 1997;390:417-421; Britton et al., Am J Physiol Heart Circ Physiol 2000;279:H2225-2233; and Duan et al. J Physiol 2001;531:437-444). Specifically known are the following: a study in which gpClC-3 clones were cloned from guinea pig hearts, expressed in the atria and ventricles by the detection using Northern blot and transfected into cultured cells (NIH/3T3 cells), which were used to detect current activity of similar nature to VSOR-ClC current found in myocardium (Duan et al. Nature 1997;390:417-421); a study in which ClC-3 specific antibodies were used to show immunohistochemically that ClC-3 existed in both sarcolemmal membranes and cytoplasmic regions (Britton et al. Am J Physiol Heart Circ Physiol 2000;279:H2225-2233); and a study in which VSOR-ClC-like currents were shown to be suppressed in gpClC-3 transfected NIH/3T3 cells by injecting them with an anti-ClC-3 antibody (product of Alomone) and which further showed that VSOR-ClC currents inherently observed in guinea-pig myocardium were similarly suppressed by injection of the same antibody, thus concluding that endogenous ClC-3 is an entity responsible for VSOR-ClC inherently observed in myocardium (Duan et al. J Physiol 2001;531:437-444).

On the other hand, it was suggested in experiments employing cells from tissues other than the myocardium that ClC-3 was not the entity of VSOR-ClC (Stobrawa et al. Neuron 2001;29:185-196; and Weylandt et al. J Biol Chem 2001;276:17461-17467). Specifically known are the following: a study in which a knockout mouse with a deletion of exon 3 from the ClC-3 gene was created for the first time and which, in view of the fact that in the knockout mouse, ClC-3 was absent from the membrane protein, there was no enhancement of ClC-4 and ClC-5 expression and that VSOR-ClC currents were not affected in hepatocytes and pancreatic acinar cells, suggested that the entity of VSOR-ClC was other than ClC-3, and which further showed that ClC-3 existed in intracellular endosomal membranes and that the homo-knockout mouse had only poor postnatal growth (weight gain), suffering from degeneration of the hippocampus and the retina (Stobrawa et al. Neuron 2001;29:185-196); and a study using HEK293 cells with stable expression of any one of several hClC-3 variants demonstrated that the ClC-3 protein which was predominantly located on intracellular organelles such as Golgi body was also found in the plasma membranes, but concluded that hClC-3 is not VSOR-ClC since both the control (untransfected) cells and a series of transfected cells showed no significant difference in VSOR-ClC current activity and RVD (Weylandt et al. J Biol Chem 2001;276:17461-17467).

Thus, it is not completely clear if VSOR-ClC can similarly suppress apoptosis in cells or tissues other than the heretofore studied cell lines, for example, in myocardial cells. In addition, little has been known about the mechanism behind apoptosis in myocardial cells and which compounds are capable of selectively suppressing apoptosis in myocardial cells.

### DISCLOSURE OF THE INVENTION

### TECHNICAL PROBLEMS TO BE SOLVED BY THE INVENTION

An object of the present invention is to provide therapeutic or prophylactic agents of cardiac disease which comprise substances capable of selectively suppressing apoptosis in cardiovascular cells.

Another object of the invention is to provide a method of screening for substances capable of selectively suppressing apoptosis in cardiovascular cells.

Yet another object of the invention is to provide therapeutic or prophylactic agents of cardiac disease which comprise compounds obtained through screening by the method and which depend for their efficacy on the suppression of apoptosis in cardiovascular cells.

### MEANS OF SOLVING THE TECHNICAL PROBLEMS

As the result of their continued intensive studies, the present inventors found that the above-stated problems could be solved.

To state briefly, it was found that the first objective of the present invention, i.e., providing therapeutic or prophylactic agents of cardiac disease, can be attained by incorporating Cl⁻ channel blockers as an active ingredient.

Using cardiovascular cells, the inventors of the present invention made studies to see if the occurrence of apoptosis could be inhibited or suppressed by administering Cl⁻ channel blockers. More specifically, the inventors induced apoptosis in cardiovascular cells and examined if the induced apoptosis could be prevented by administering the cells with Cl⁻ channel blockers under test. As a result, it became clear that the administered Cl⁻ channel blockers inhibited the mechanism of apoptosis in cardiovascular cells to successfully suppress apoptosis occurring in the cells. The present invention has been accomplished on the basis of this finding.

The term "Cl⁻ channel blockers" as used herein means substances that inhibit the Cl⁻ transport via Cl⁻ channels and they include, for example, low-molecular weight compounds, antibodies, antisense compounds, etc. that have the Cl⁻ channel inhibitory effect. In the present invention, the Cl⁻ channel blockers are preferably VSOR-ClC blockers and, hence, low-molecular weight compounds, antibodies, antisense compounds, etc. that have VSOR-ClC inhibitory effect are preferred. More preferred examples of Cl⁻ channel blockers include low-molecular weight compounds, antibodies, antisense compounds, etc. that specifically inhibit ClC-3 which is VSOR-ClC.

Low-molecular weight compounds that can be used in the invention as VSOR-ClC blockers include, but not limited to, 4-acetamido-4'-isothiocyanostilbene (SITS), 4,4'-diisothiocyanostilbene-2,2'-disulfonic acid (DIDS), 5-nitro-2-(3-phenylpropylamino)-benzoate (NPPB), phloretin, niflumic acid, glibenclamide, fluoxetine, tamoxifen, clomiphene and nafoxidine, as well as the compounds described in the following papers: DDT 2000;5:492-505; Br J Pharmacol 1999;126:508-514; and Br J Pharmacol 2001;132:135-142. However, any other compounds will do if they have the action of VSOR-ClC blocking. To state more specifically, particularly preferred are Cl⁻ channel blockers that have high selectivity for VSOR-ClC but low selectivity for other Cl⁻ channels such as the cAMP-activated Cl⁻ channel (CFTR) and the Ca²⁺-activated Cl⁻ channel (CaCC), the properties possessed by SITS, DIDS and phloretin, among which SITS and DIDS are most preferred.

From the low-molecular weight compounds listed above, those which are capable of suppressing apoptotic cell death in cardiovascular cells can be chosen and used as therapeutic or prophylactic agents of cardiac disease in the present invention.

Antibodies that can be used in the invention as VSOR-ClC blockers are not limited in any particular way as long as they bind to VSOR-ClC to inhibit its function and mouse antibodies, rat antibodies, rabbit antibodies, sheep antibodies, chimeric antibodies, humanized antibodies, human antibodies, etc. can be employed as appropriate. The antibodies may be polyclonal or monoclonal but monoclonal antibodies are preferred since homogeneous antibodies can be produced consistently. Polyclonal and monoclonal antibodies can be prepared by methods well known to the skilled artisan.

In order to prepare such antibodies, the Cl⁻ channel proteins described herein may be used as immunogens. More preferably, VSOR-ClC proteins are used as immunogens, and among VSOR-ClC proteins, the ClC-3 protein is most preferred.

Those antibodies are prepared by immunizing animals such as mouse, rat, rabbit and goat with the above-mentioned immunogens. For immunizing, immunogens may be administered together with adjuvants in order to potentiate the immune activity of the animal where the antibody is to be produced. For examples of applicable adjuvants, reference may be had to Martin, REMINGTON'S PHARM. SCI., 15th Ed. (Mack Publ. Co., Easton (1975)). Any adjuvants may be used as chosen from water-in-oil emulsions, oil-in-water emulsions, aluminum adjuvants and so on. Exemplary water-in-oil emulsions include Freund's Complete Adjuvant and Freund's Incomplete Adjuvant; exemplary oil-in-water emulsions include the RIBI Adjuvant System (RIBI Immunol. Res. Inc.); exemplary aluminum adjuvants include aluminum potassium sulfate; however, these are not the sole examples that can be used in the invention.

After several immunizations, a small amount of blood is taken from the immunized animal and checked for the actual production of an antibody characterized by its substantial ability to bind specifically to the Cl⁻ channel protein. In the case where the intended antibody has been produced in the blood of the immunized animal, the animal is sacrificed and spleen cells are isolated and fused with myeloma cells to make hybridoma cells.

Hybridomas that produce monoclonal antibodies can basically be prepared in the following manner using known techniques. Briefly, the desired antigen itself or cells that express the desired antigen are used as a sensitized antigen and immunization is performed in accordance with ordinary immunizing procedures and the obtained immunocytes are fused to known parent cells by an ordinary cell fusing method; the fusion cells are screened by ordinary procedures to obtain monoclonal antibody-producing cells (hybridomas), which are tested by screening to check if the antibody produced by those cells will exhibit a reaction specific to the immunogen. Hybridomas can be prepared in accordance with known procedures, say, the method of Milstein et al. (Kohler, G. and Milstein, C., Methods of Enzymol. (1981) 73:3-46). If the antigen has only low immunogenicity, immunization may be performed after binding it to an immunogenic macromolecule such as albumin, KLH, etc. Among the hybridoma cells obtained by the method described above, clones are made of those hybridoma cells which can produce antibodies that bind specifically to the target Cl⁻ channel protein.

If desired, an antibody gene may be cloned from hybridomas, incorporated into a suitable vector, which is introduced into a host and processed by gene recombinant technology to produce a gene recombinant antibody (see, for example, Carl, A.K. Borrebaeck and James, W. Larrick, THERAPEUTIC MONOCLONAL ANTIBODIES, Published in the United Kingdom by MACMILLAN PUBLISHERS LTD., 1990). Specific procedures are as follows: from the mRNA of the hybridomas, cDNA for the variable (V) region of an antibody is synthesized using a reverse transcriptase; when DNA coding for the V region of the intended antibody is obtained, it is linked to DNA coding for a desired antibody's constant (C) region and incorporated into an expression vector. Alternatively, DNA coding for the V region of the antibody may be incorporated into an expression vector containing the DNA of the antibody's C region. DNA should be incorporated into the expression vector such that it will be expressed under the control of an expression regulatory region such as an enhancer or a promoter. In the next step, the host cell is transformed with the expression vector to express the intended antibody.

In the present invention, modified artificially gene recombinant antibodies for certain purposes such as reducing heteroantigenicity to humans may be employed and their examples include chimeric antibodies and humanized antibodies. Such modified antibodies can be produced by known procedures. Chimeric antibodies are those antibodies which consist of the variable region of light and heavy chains in an antibody from a non-human mammal such as mouse and the constant region of light and heavy chains in a human antibody. The chimeric antibody can be produced by linking the DNA coding for the variable region of the mouse antibody to the DNA coding for the constant region of the human body, incorporating the linked DNAs into an expression vector and then introducing the vector into a host to produce it (WO 86/01533).

Humanized antibodies which are also called reshaped human antibodies are prepared by putting the complementarity determining region (CDR) of an antibody from a non-human mammal such as mouse into the complementarity determining region of a human antibody, and general procedures of gene recombinant technology for obtaining humanized antibodies are also known. To state specifically, a DNA sequence designed to link the CDR of a mouse antibody to the framework region (FR) of a human antibody is synthesized by PCR using several oligonucleotides so prepared as to have overlaps in their terminal portions; the obtained DNA is linked to the DNA coding for the human antibody's constant region and the linked DNAs are incorporated into an expression vector, which is then introduced into a host (see EP 239400 and WO 96/02576). The FR of the human antibody to be linked via CDR is so selected that the complementarity determining region will form a satisfactory antigen binding site. Depending on the need, amino acids in the framework region of the variable region of the reshaped human antibody may be replaced in such a way that the complementarity determining region of the reshaped human antibody will have a suitable antigen binding site (Sato, K. et al., Cancer Res. (1993) 53, 851-856).

Methods of obtaining human antibodies are also known. For example, human lymphocytes are sensitized in vitro with a desired antigen or cells that express the desired antigen and the sensitized lymphocytes are fused to human myeloma cells such as U266 to make a desired human antibody having binding activity to the antigen (see JP 59878/89 B). A desired human antibody can also be obtained by immunizing with an antigen a transgenic animal having the whole repertoire of the human antibody's genes (see WO 93/12227, WO 92/03918, WO 94/02602, WO 94/25585, WO 96/34096 and WO 96/33735). A technique is known that employs a human antibody library to acquire a human antibody by the panning method. For example, the variable region of a human antibody is expressed on the surfaces of phages as a single-chained antibody (scFv) by the phage display method and phage that binds to the antigen is selected. By analyzing the gene of the selected phage, a DNA sequence coding for the human antibody binding to the antigen can be determined. Once the DNA sequence of the scFv that binds to the antigen has been identified, that sequence is introduced into a suitable expression vector to obtain the intended human antibody. These methods are known and reference may be had to WO 92/01047, WO 92/20791, WO 93/06213, WO 93/11236, WO 93/19172, WO 95/01438 and WO 95/15388.

In the case of isolating an antibody's gene before it is introduced into a suitable host to prepare an antibody, suitable combinations of host and expression vector may be employed. If eukaryotic cells are the host, they may be animal cells, plant cells or fungal cells. Known animal cells include: (1) mammalian cells such as CHO, COS, myeloma, BHK (baby hamster kidney), HeLa and Vero; (2) amphibian cells such as *Xenopus* oocytes; and (3) insect cells such as sf9, sf21 and Tn5. Known plant cells include cells derived from a species of the genus *Nicotiana*, say, *Nicotiana tabacum* and they may be subjected to callus culture. Known fungal cells include yeasts (e.g. a species of the genus *Saccharomyces*, say, *Saccharomyces serevisiae*) and filamentous fungi (e.g. a species of the genus *Aspergillus*, say, *Aspergillus niger*). If prokaryotic cells are the host, one may employ bacterial cells in the production system. Known bacterial cells are *E. coli* and *B. subtilis*. An intended antibody's gene is introduced into those cells by transformation and the transformed cells are cultured in vitro to produce the desired antibody.

Antisense compounds can also be used in the invention as VSOR-C1C blockers (O'Connor, J Neurochem (1991) 56:560 in Oligodeoxynucleotides as Antisense Inhibitors of Gene Expression, CRC Press, Boca Raton, FL (1988)). One may also employ oligonucleotides that form triple helices with the gene of VSOR-ClC (Lee et al., Nucleic Acids Res (1979) 6:3073; Cooney et al., Science (1988) 241:456; and Dervan et al., Science (1991) 251:1360).

Such antisense compounds and oligonucleotides can be prepared on the basis of DNAs that encode those proteins such as rat ClC-3 (Kawasaki M et al., Neuron 1994;12:597-604) and human ClC-3 (GenBank Accession No. AF172729 and Huang P et al., J. Biol. Chem. 2001;276, 20093-20100) which are considered the target of VSOR-ClC blockers of the present invention. Specifically, the above-described antisense compounds or oligonucleotides can be prepared in such a way that they are capable of binding to nucleotide sequences such as the promoter region, exon region and translation termination region of those genes.

It is possible to verify by conducting an experiment either *in vivo* or *in vitro* or both *in vivo* and *in vitro* whether Cl⁻ channel blockers including the above-described low-molecular weight compounds, antibodies, antisense compounds and oligonucleotides can be used as therapeutic and/or prophylactic agents of cardiac disease. Specifically, verification can be made *in vivo* by an experiment employing a living model such as an ischemia/reperfusion model, and *in vitro* verification can be made by screening in which low-molecular weight compounds, antibodies, antisense compounds or oligonucleotides that are not known in terms of function are checked for their ability to actually suppress apoptotic cell death in cardiovascular cells. Screening is preferably performed *in vitro* in the present invention.

Thus, the second objective of the present invention is attained by providing a method for screening the above-described low-molecular weight compounds, antibodies, antisense compounds and oligonucleotides to see if they have the Cl⁻ channel inhibiting effect of the invention and can suppress apoptotic cell death in cardiovascular cells.

The screening method of the invention is characterized by comprising the steps of inducing apoptosis in cardiovascular cells, treating the cells with a Cl⁻ channel blocker under test, and evaluating the therapeutic and/or prophylactic effect of the blocker under test on cardiac disease by checking to see if it can suppress apoptotic cell death in cardiovascular cells.

In one embodiment of the present invention, apoptosis is induced in cardiovascular cells and simultaneously with or subsequent to this induction of apoptosis, said cardiovascular cells are treated with a compound under test. Check is made to see if apoptotic cell death is suppressed in the cardiovascular cells in the presence of the compound under test as compared with the case where no such compound is present. In this way, one can screen to see if the compound under test proves the therapeutic and/or prophylactic effect on cardiac disease by suppressing apoptotic cell death in cardiovascular cells.

Examples of the cardiovascular cells contemplated in the invention include myocardial cells, vascular endothelial cells, vascular smooth muscle cells, fibroblasts, myofibroblasts, pericytes and vascular endothelial progenitor cells, with myocardial cells and vascular endothelial cells being preferred. These can be used either as primary cultures of cells or as cells derived from cell lines. If primary cultures of cells are used as cardiovascular cells, heart and/or blood vessels are taken from animals such as rat, mouse, guinea pig, rabbit, bovine and horse for which primary culture systems have been established. The sampled heart and/or blood vessels are treated with a proteolytic enzyme such as collagenase or trypsin before they are prepared as cell suspensions of a predetermined cell density. If cells derived from cell lines are used as cardiovascular cells, either one of the following generally applicable cell lines can be employed, i.e., myocardial cell line, vascular endothelial cell line, vascular smooth muscle cell line, fibroblast cell line, myofibroblast cell line, pericyte cell line and vascular endothelial progenitor cell line, with myocardial cell line and vascular endothelial cell line being preferred. In the case under consideration, the cell line to be employed is cultured under suitable conditions, then prepared as a cell suspension of a predetermined cell density.

Methods for inducing apoptosis in cardiovascular cells in the present invention include, but are not limited to, chemical methods employing staurosporine (STS), tumor necrosis factor (TNF), TNF in combination with cycloheximide (CHX), anti-Fas agonist antibody, anticancer agent, hydrogen peroxide, etc.; biological methods employing viruses; physical methods employing ultraviolet light, radiations, warm heat, etc.; and combinations of these methods. The dose and the time of treatment required to induce apoptotic cell death in the above-listed cardiovascular cells depend on the cell to be treated and the substance employed to induce apoptosis and can be appropriately determined by the skilled artisan. If staurosporine is employed, treatment is done at a concentration of 0.3-3 µM for 10 minutes to 24 hours, preferably for 30 minutes to 8 hours, to induce apoptosis; if a mixture of TNF and CHX is employed, 0.1-1 µg/ml of CHX is added to 2-10 ng/ml of TNFα and treatment with the mixture is done for 10 minutes to 24 hours, preferably for 30 minutes to 8 hours.

In the screening method of the invention, the compound under test may be added simultaneously with the above-described induction of apoptosis or, alternatively, it may be added before or after apoptogenic stimulation by the above-described induction of apoptosis. In order to verify its efficacy and effective dose, the compound under test is tested at several concentrations. It is generally preferred to perform an experiment with the concentration of the compound under test being varied over a range from about 10 µM to about 10 mM. The time period of cell treatment with the compound under test depends on the cell to be treated and can be appropriately determined by the skilled artisan; generally speaking, 2-24 hour treatment is sufficient to test for the apoptosis suppressing effect of the compound under test.

In the present invention, the compound under test can be evaluated for the apoptosis suppressing effect by checking to see if the cell treated by the compound shows any characteristics of apoptosis. Specific characteristics of apoptosis include, for example, decreased cell volume, cytochrome c release, caspase (preferably, caspase-3) activation, DNA fragmentation, morphological features such as the formation of apoptotic bodies, physiological changes such as the expression of an apoptosis-related specific antigen, and decreased cell viability. These characteristics can be detected by the following measurements.

Cell volume can be determined by an electronic size determining technique using a Coulter cell size analyzer (CDA-500; Sysmex, Kobe, Japan) (Hazama, A. and Okada, Y., J. Physiol., 402, 687-702, 1988).

Cytochrome c is released from mytochondria and can be studied with a confocal laser scanning fluorescence microscope (e.g. Bio-Rad MRC-1024). In this case, cytochrome c can be labelled with a fluorescently labelled anti-cytochrome c monoclonal antibody [e.g. 6H2.B4 (BD PharMingen, San Diego, CA, USA)] (Deshmukh, M. and Johnson, E.M. Jr., Neuron, 21, 695-705, 1988). Release of cytochrome c can also be detected by analyzing cytoplasmic fractions by Western blot. In this case, cytoplasmic fractions may be electrophoresed on a polyacrylamide gel before cytochrome c is detected with an anti-cytochrome c monoclonal antibody [e.g. 7H8.2C12 (BD PharMingen, San Diego, CA, USA] (Liu, X. et al., Cell, 86, 147-157, 1996).

The activity of caspase-3 can be detected by fluorometry. In order to exclude involvement of any other related proteases, the difference in the intensity of fluorescence between the absence and the presence of a specific inhibitor of caspase-3 is preferably detected. A fluorescing substrate labelled with fluorochrome 7-amino-4-methylcoumarin (AMC) was added to a CaspASE assay system (SIGMA CHEMICAL Co., St. Louis, MO, USA or Promega, Madison, WI, USA) in order to detect caspase-3 (Ac-DEVD-AMC) and a tetrapeptide inhibitor of caspase-3 (Ac-DEVD-CHO).

Internucleosomal fragmentation of DNA can be detected as DNA ladder (Shiokawa, D. et al., Eur. J. Biohem., 226, 23-30, 1994). Summarily, 37°C x 1 hr treatment in a lysing buffer (10 mM EDTA/0.5% Na-N-lauroyl sarcosinate/500 µg/ml RnASE/50 mM Tris·HCl, pH 7.8) was followed by 37°C x 1 hr treatment with 500 µg/ml of proteinase K to lyse the cells. Chromosomal DNA was analyzed by agarose gel electrophoresis (2%) and later stained with ethidium bromide.

DNA fragmentation can also be detected by techniques of flow cytometry such as modified TUNEL method (TdT assay) and Sub-G1 measurement. In modified TUNEL method (TdT assay), intracellular DNA fragments are FITC labelled by TUNEL method, the total DNA is stained with PI (propidium iodide), and the fluorescence derived from both fluorescence, i.e., FITC and PI, are measured simultaneously by flow cytometry to detect the degree of DNA fragmentation. In Sub-G1 measurement, DNA fragments are washed out of the cells and apoptotic cells are identified by flow cytometry as cells having a smaller DNA content than live G1-phase cells (i.e. as sub-G1 cells).

At the early stage of apoptosis, phosphatidylserine, phosphatidylethanolamine, etc. migrate to the surface of cell membrane and become exposed to the extracellular environment; these can be detected by flow cytometry using Annexin V. They can also be detected by another technique of flow cytometry such as measurement of mitochondrial membrane potential.

Transmission electron microscopy of cells was performed with JEM 100CX (Tokyo, Japan). Cultured cells were prefixed in a Karnovsky fixing solution using a CaCl₂-free 0.1 M Na-phosphate buffer. After osmification with 1% OsO₄ in water, the cells were dehydrated in stepwise ethanol gradient and Epon embedded.

Viability of cells cultured on 24- or 96-well multiwell plates was evaluated by mitochondrial dehydrogenase activity using the colorimetric MTT assay. Colorimetric MTT assay depends on the fact that viable cells can reduce 3-(4,5-dimethyl-2-thiazolyl)-2,5-diphenyl-2H-tetrazolium bromide (MTT) but dead cells cannot.
Instead of MTT, improved formazan reagents such as WST-1 or WST-8 may be substituted. Detection may be performed using the Cell Counting Kit (DOJINDO LABORATORIES, Kumamoto, Japan) or Cell Counting Kit-8 (DOJINDO LABORATORIES, Kumamoto, Japan). Cell viability can also be assessed by trypan blue exclusion from viable cells after 5-min incubation with 0.4% trypan blue.

Other methods that can detect apoptosis include TUNEL method, ISEL method, hematoxylin-eosin (HE) staining, immunohistochemical staining and electron microscopy, all applied to tissue specimens.

The present invention attains its third objective by providing therapeutic and/or prophylactic agents of cardiac disease that contain the Cl⁻ channel blockers as selected by the above-described screening method.

The therapeutic and/or prophylactic agents of cardiac disease according to the invention can serve their purpose by suppressing apoptosis in myocardial cells on account of the inhibition of the Cl⁻ channel by the Cl⁻ channel blockers as chosen by the above-described screening method.

The dosage of the Cl⁻ channel blocker contained in the therapeutic and/or prophylactic agents of cardiac disease, as well as the method and frequency of its administration can be easily determined by the skilled artisan considering various factors including the sex, age, body weight and the systemic condition of the subject to whom it is administered. The dose as measured in terms of the active ingredient is generally within the range from 0.001 to 1000 µg/kg/day, preferably from 0.01 to 100 µg/kg/day, more preferably from 0.1 to 10 µg/kg/day. If the active ingredient is an antibody with inhibitory action of VSOR-ClC function, the dose as measured in terms of the active ingredient is generally within the range from 0.001 to 1000 mg/kg/day, preferably from 0.1 to 50 mg/kg/day, more preferably from 0.5 to 10 mg/kg/day. However, the therapeutic and/or prophylactic agents of cardiac disease according to the invention are by no means limited to those dosage levels.

The dosing schedule including the dosing period depends on the severity of the disease to be treated, its responsiveness to the treatment and the accumulation of the drug in the patient's body and administration can be continued for several days to several months, sometimes over several years, or until the treatment proves effective or until amelioration of the diseased condition or prevention of the risk of disease is accomplished, at a frequency of one to three times a day, or at least once a week, month or year.

The therapeutic and/or prophylactic agents of cardiac disease according to the invention can be administered in various treatments irrespective of whether they are topical or systemic. Administration may be conducted orally or parenterally. Practices of parenteral administration include, for example, intravenous drip, catheterized intracardiac administration or administration to an intended site of blood vessel, intraperitoneal injection and intramuscular injection.

Therapeutic and/or prophylactic agents of cardiac disease that are intended for oral administration include powders or granules, suspensions or solutions in aqueous or nonaqueous media, capsules, sachets, and tablets. Thickeners, flavoring agents, diluents, emulsifiers, dispersing aids and binders may be desirable to be included. Therapeutic and/or prophylactic agents for cardiac disease that are intended for parenteral administration may include sterilized aqueous solutions containing buffers, diluents or other suitable additives.

The therapeutic and/or prophylactic agents for cardiac disease according to the invention may contain any additives that are suitable for administering them to cardiovascular cells, as exemplified by pharmaceutically acceptable carriers, thickeners, diluents, buffers, preservatives, surfactants, neutral or cationic lipids, lipid complexes, liposomes, penetration enhancers, carrier compounds and other pharmaceutically acceptable carriers and vehicles. As penetration enhancers for enhancing alimentary transport of therapeutic and/or prophylactic agents of cardiac disease, either one of the members of the group consisting of fatty acids, bile acid salts, chelating agents, surfactants and non-surfactants may be incorporated.

Pharmaceutically acceptable carriers include, but are not limited to, binders (e.g. pregelatinized corn starch, polyvinylpyrrolidone and hydroxypropyl methylcellulose); fillers (e.g. lactose and other sugars, microcrystalline cellulose, pectin, gelatin, calcium sulfate, ethyl cellulose, polyacrylate and calcium hydrogen phosphate); lubricants (e.g. magnesium stearate, talc, silica, colloidal silicon dioxide, stearic acid, metallic stearates, hydrogenated vegetable oils, corn starch, polyethylene glycol, sodium benzoate and sodium acetate); disintegrants (e.g. starch); and wetting agents (e.g. sodium lauryl sulfate).

The therapeutic and/or prophylactic agents of cardiac disease according to the invention may additionally contain ancillary components to the extent that will not impair the biological activity of the Cl⁻ channel blockers of the invention. For example, the therapeutic and/or prophylactic agents of cardiac disease according to the invention may contain additional substances useful for formulating the therapeutic and/or prophylactic agents of cardiac disease according to the invention and examples of such additional substances include pigments, flavoring agents, preservatives, antioxidants, opacifiers, thickeners and stabilizers.

### BRIEF DESCRIPTION OF THE DRAWINGS

Fig. 1 is a graph showing how SITS was effective at 125, 250 and 500 µM in suppressing the 1 µM staurosporine (STS-1) induced decrease in viability of primary cultures of rat myocardial cells (cultured rat myocardial cells) on 24-well multiwell plates; each column represents a mean value and each bar, ± standard error (SE), and these definitions will apply in the following figures;
Fig. 2 is a graph showing how SITS was effective at 500 and 1000 µM in suppressing the STS-1 induced decrease in viability of cultured rat myocardial cells on 24-well multiwell plates;
Fig. 3 is a graph showing how SITS was effective at 250 and 500 µM in suppressing the STS-1 induced decrease in viability of cultured rat myocardial cells on 96-well multiwell plates;
Fig. 4 is a graph showing how DIDS was effective at 62.5, 125 and 250 µM in suppressing the STS-1 induced decrease in viability of cultured rat myocardial cells on 24-well multiwell plates;
Fig. 5 is a graph showing how DIDS was effective at 250 µM in suppressing the STS-1 induced increase in the caspase-3 activity of cultured rat myocardial cells on 12-well multiwell plates;
Fig. 6 is a graph showing that even in an HCO₃⁻-free culture solution, the STS-1 induced a decrease in the viability of cultured rat myocardial cells on 24-well multiwell plates and also showing how DIDS was effective at 62.5, 125 and 250 µM in suppressing the STS-1 induced decrease in cell viability;
Fig. 7 is a graph showing that even in an HCO₃⁻-free culture solution, the STS-1 induced a decrease in the viability of cultured rat myocardial cells on 96-well multiwell plates and also showing how SITS was effective at 125, 250 and 500 µM in suppressing the STS-1 induced decrease in cell viability;
Fig. 8 is a graph showing how SITS was effective at 125, 250 and 500 µM in suppressing the STS-1 induced decrease in viability of a cell line from bovine arterial endothelial cells on 24-well multiwell plates; and
Fig. 9 is a graph showing how SITS was effective at 500 µM in suppressing the 1 µM or 3 µM staurosporine (STS-1 or STS-3) induced decrease in viability of a cell line from bovine arterial endothelial cells on 24-well multiwell plates.

### BEST MODES FOR CARRYING OUT THE INVENTION

In one embodiment of the invention, with primary cultures of rat myocardial cells being used as cardiovascular cells, tests were conducted to assess the apoptosis inducing effect of staurosporine and the effectiveness of SITS (4-acetamido-4'-isothiocyanostilbene) and DIDS (4,4'-diisothiocyanostilbene-2,2'-disulfonic acid) in protecting those cells from apoptosis.

During cultivation, all cell manipulations were performed under sterile conditions.

The myocardial cells used in the experiments were those of primary cultures from rats. In order to prepare primary cultures of myocardial cells, heart was excised from SD rats at day 20 of pregnancy (purchased from Japan SLC Co., Ltd.) and enzyme-digested with collagenase (Worthington Biochemical Corp.) and trypsin (GIBCO BRL, Gaithersberg, MD) to isolate myocardial cells and make a cell suspension of them. As an ordinary culture medium, D-MEM supplemented with fetal bovine serum (FBS) to give a concentration of 10% (GIBCO BRL, Gaithersberg, MD) was employed. For checking to see if Cl⁻/HCO₃⁻ anion exchangers (AEs) would be involved in apoptosis in cardiovascular cells and in protection from it, Leibovitz's L-15 Medium supplemented with FBS to give a concentration of 10% (GIBCO BRL, Gaithersberg, MD) was employed as an HCO₃⁻-free culture medium and the result from it was compared with the result from the ordinary culture medium. The HCO₃⁻-free culture medium was employed in order to suppress the function of the Cl⁻/HCO₃⁻ anion exchangers by depleting HCO₃⁻. The Cl⁻/HCO₃⁻ anion exchangers are widely distributed in erythrocytes and other tissues, AE1 being expressed primarily in erythrocytes and AE3 in myocardial cells and neurons. By coupling the exclusion of intracellular Cl⁻ to the outside of cells and intake of extracellular HCO₃⁻ into the cells, the exchangers function as an intracellular Cl⁻ and pH regulating system and they also work as a cell volume regulating system during regulatory volume increase (RVI). In a pathological context, it is speculated that the exchangers participate in the process of myocardial cells' acidification which accompanies ischemia.

A suspension of myocardial cells at a density of 3-5 x 10⁵ cells/mL was dispensed in 24-well multiwell plates (Falcon) in an amount of 0.5 mL per well, or in 96-well multiwell plates (Falcon) in an amount of 0.1 mL per well, or in 12-well multiwell plates (Falcon) in an mount of 1 mL per well. The plates were placed in a CO₂ incubator and cultured in 5% CO₂ at 37°C for 2-3 days. The thus cultured cells were used in subsequent experiments.

A control group consisted of three culture media, a cell-free medium (blank), a cell-containing medium alone (D-MEM) and 0.3% DMSO [D-MEM supplemented with 0.3% DMSO (DOJINDO LABORATORIES, Kumamoto, Japan)]. A test group consisted of three culture media containing a drug or drugs under specified conditions, i.e., a solution of 1 µM staurosporine (Sigma) dissolved in 0.3% DMSO, a mixed solution of the 1 µM staurosporine and 125-1000 µM SITS (SIGMA CHEMICAL Co., St. Louis, MO, USA), and a mixed solution of the 1 µM staurosporine and 62.5-250 µM DIDS (SIGMA CHEMICAL Co., St. Louis, MO, USA); the culture media of the test group are hereunder referred to as "conditioned culture media".

Specifically, after aspirating away a culture medium, a fresh medium of the control group or a conditioned culture medium of interest was added in 24-well multiwell plates in an amount of 0.5 mL/well. After adding the conditioned culture medium, the plates were placed in a CO₂ incubator and cultured in 5% CO₂ at 37°C for 2-4 hours. When a conditioned culture medium based on the HCO₃⁻-free culture medium was added, the plates were placed in an atmospheric incubator and cultured at 37°C. Thereafter, the conditioned culture solution was aspirated away and in order to ensure that no conditioned culture medium would remain, the wells were washed once with PBS(-) in amounts of 0.5-0.7 mL/well and the washings were aspirated away, followed by adding the ordinary culture medium (D-MEM supplemented with 10% FBS) in an amount of 0.5 mL/well. The same procedures were repeated on 96-well multiwell plates using volumes that were each a fifth of the amounts specified above and on 12-well multiwell plates using volumes of 1 mL.

The thus prepared cells were reacted with WST-8 [2-(2-methoxy-4-nitrophenyl)-3-(4-nitrophenyl)-5-(2,4-disulfophenyl)-2H-tetrazolium monosodium salt] as a fluorescing substrate (Cell Counting Kit-8 of DOJINDO LABORATORIES, Kumamoto, Japan) to measure the cell viability.

More specifically, in the case of 24-well multiwell plates, a solution of Cell Counting Kit-8 was added to the culture medium in an amount of 0.05 mL/well and the plates were replaced in the CO₂ incubator and cultured in 5% CO₂ at 37°C for appropriate periods between 1 to 4 hours, mostly for 2 hours. Thereafter, the culture medium was sampled in an amount of 0.11 mL from each well and dispensed among specified positions on 96-well multiwell plates. In the case of 96-well multiwell plates, a solution of Cell Counting Kit-8 was added to the culture solution in an amount of 0.01 mL/well and the plates were cultured in the same way as in the case of the 24-well multiwell plates. The cultured plates were loaded on a microplate reader (Bio-Rad, Model 3550, Bio-Rad Laboratories, Hercules, CA, USA) to measure the absorbance at 450 nm (reference wavelength: 655 nm), which was used as an index of viable cell count.

The caspase-3 activity of the thus prepared cells was measured with Caspase-3 Assay Kit, Fluorimetric (SIGMA CHEMICAL, Co., St. Louis, MO, USA).

More specifically, 12-well multiwell plates were cultured for 2 hours after adding a conditioned culture solution, which was then removed and further cultivation was effected for an appropriate period, mostly for 2 hours; thereafter, cell lysates were prepared using Caspase-3 Assay Kit, Fluorimetric; in the subsequent stage, the amount of formation of specific fluorochrome 7-amino-4-methylcoumarin (AMC) was measured by means of a fluorometric microplate reader (SPECTRA Fluor, Wako Pure Chemical Industries, Ltd., Osaka, Japan) according to the procedure of measurement with the Kit; and the amount of AMC formation was used as an index of caspase-3 activity.

In another embodiment of the invention, a cell line from bovine arterial endothelial cells was employed as cardiovascular cells to test the apoptosis inducing effect of staurosporine and the effectiveness of SITS in protecting the cells from apoptosis.

Cultured cells derived from a bovine arterial endothelial cell line were used as endothelial cells. Cultured cells derived from an endothelial cell line were prepared from a bovine vascular (arterial) endothelium-derived cell line (GM07372). The culture medium was D-MEM supplemented with 10% of fetal bovine serum (FBS) (GIBCO BRL, Gaithersberg, MD).

A suspension of endothelial cells at a density of 5 x 10⁴ cells/mL was dispensed in 24-well multiwell plates in an amount of 0.5 mL per well. The plates were placed in a CO₂ incubator and cultured in 5% CO₂ at 37°C for 2 days. The thus cultured cells were used in subsequent experiments.

In the tests, cell viability was assessed by repeating the procedures for the primary cultures of rat myocardial cells, except for the experiments using 125-500 µM SITS.

The present invention is described below more specifically by means of examples that are provided here for illustrative purposes only and are by no means intended to limit the technical scope of the invention.

### EXAMPLES

### Example 1: The protective effect of SITS (4-acetamido-4' isothiocyanostilbene) on staurosporine-induced cytotoxicity in cultured myocardial cells

Primary cultures of myocardial cells from SD rats at day 20 of pregnancy were used after being cultivated in D-MEM supplemented with 10% of fetal bovine serum. The myocardial cells were cultured on 24-well multiwell plates in an amount of 0.5 mL per well so as to give a density of 5 x 10⁵ cells/mL.

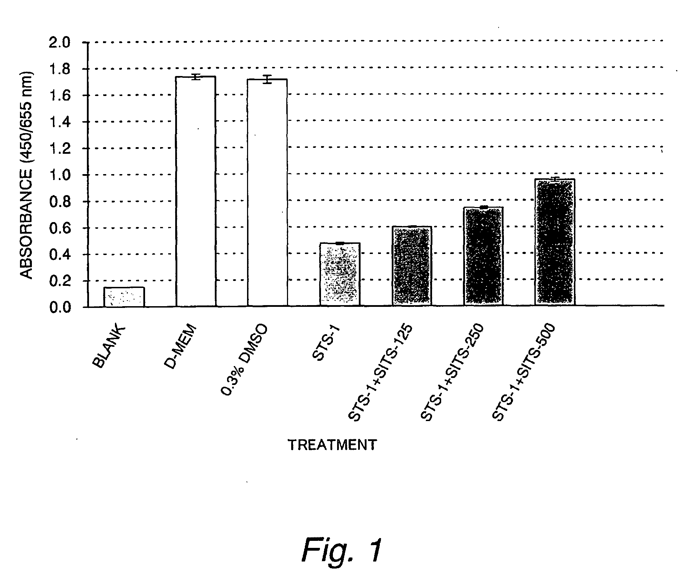

A control group consisted of three culture media, a cell-free medium (blank), a cell-containing medium alone (D-MEM) and 0.3% DMSO. A test group consisted of two drug-containing culture media, i.e., a solution of 1 µM staurosporine dissolved in 0.3% DMSO (STS-1) and a mixed solution of the 1 µM staurosporine and 125, 250 or 500 µM SITS (STS-1+SITS-125, STS-1+SITS-250, or STS-1+SITS-500). The two groups were cultured in 5% CO₂ at 37°C for 2 hours. Cultivation was continued for another day in D-MEM supplemented with 10% FBS and cell viability was measured by WST-8. The result is shown in Fig. 1.

As it turned out, it was elucidated the decrease in cell viability due to staurosporine can be suppressed by SITS in a dose-dependent manner regardless of the duration of the treatment.

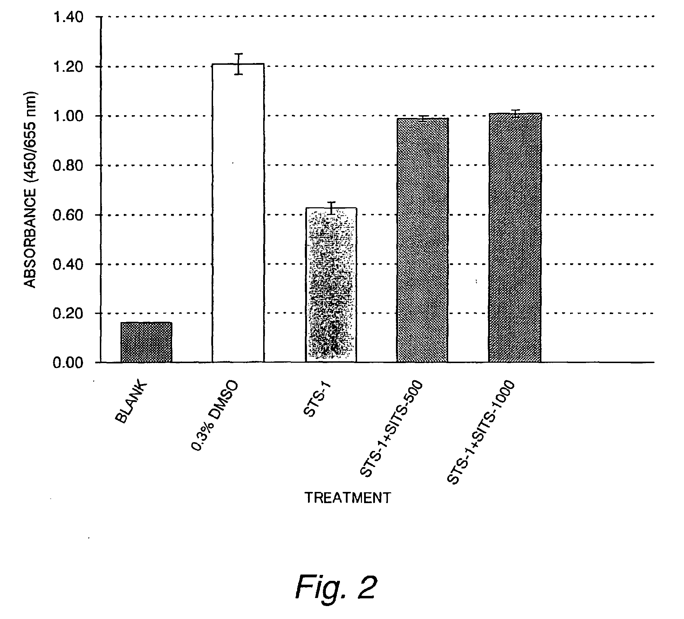

For thorough verification of the dose-dependency of the SITS ability to suppress the staurosporine-induced decrease in cell viability, similar experiments were conducted with a control group consisting of a cell-free culture medium (blank) and 0.3% DMSO alone, as well as a test group consisting of a solution of 1 µM staurosporine (STS-1) dissolved in 0.3% DMSO and a mixed solution of the 1 µM staurosporine and 500 or 1000 µM SITS (STS-1+SITS-500 or STS-1+SITS-1000); the two groups were cultured in 5% CO₂ at 37°C for 2 hours. The result is shown in Fig. 2.

As it turned out, in the case of 2-hour stimulation with staurosporine + SITS added at concentrations of 500 µM and more, it was elucidated that these concentrations of SITS exerts substantially equal levels of effectiveness in suppressing the drop in cell viability at the SITS concentrations tested.

### Example 2: The protective effect of SITS on staurosporine-induced cytotoxicity in cultured myocardial cells

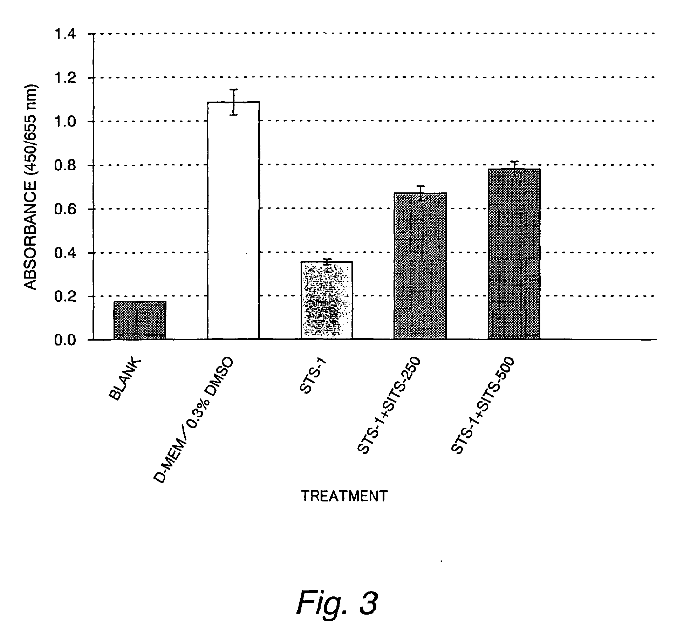

In this Example, tests were conducted as in Example 1 except that the test group consisted of a 1 µM staurosporine solution (STS-1) and a mixed solution of the 1 µM staurosporine solution and 250 or 500 µM SITS (STS-1+SITS-250 or STS-1+SITS-500) and that the control and test groups were cultured on 96-well multiwell plates rather than the 24-well multiwell plates. The result is shown in Fig. 3.

As it turned out, it was elucidated that SITS suppresses the drop in cell viability in a dose-dependent manner even when the 96-well multiwell plates were substituted for the 24-well multiwell plates. This shows the possibility of performing high throughput screening of candidate compounds by employing the conditions of Example 2.

### Example 3: The protective effect of DIDS (4,4'-diisothiocyanostilbene-2,2'-disulfonic acid) from staurosporine-induced cytotoxicity in cultured myocardial cells

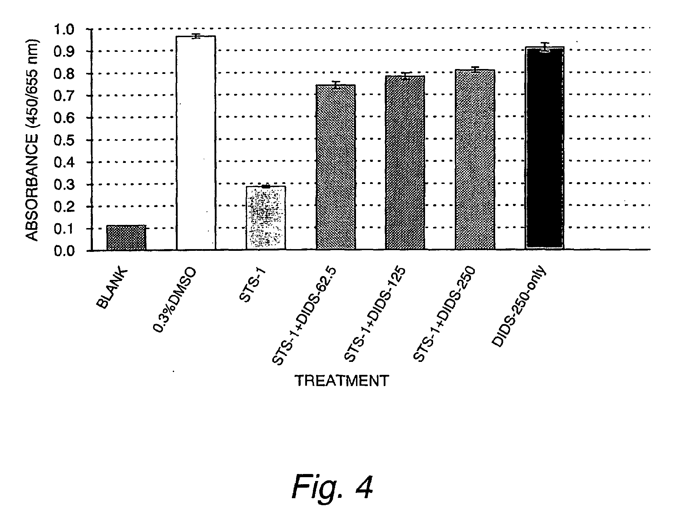

In this Example, tests were conducted as in Example 1 except that the test group consisted of a 1 µM staurosporine solution (STS-1) and a mixed solution of the 1 µM staurosporine and 62.5, 125 or 250 µM DIDS (STS-1+DIDS-62.5, STS-1+DIDS-125 or STS-1+DIDS-250). The result is shown in Fig. 4.

As it turned out, it was elucidated that DIDS instead of SITS also suppresses the drop in cell viability in a dose-dependent manner.

### Example 4: The protective effect of DIDS on staurosporine-induced increase in the caspase-3 activity of cultured myocardial cells

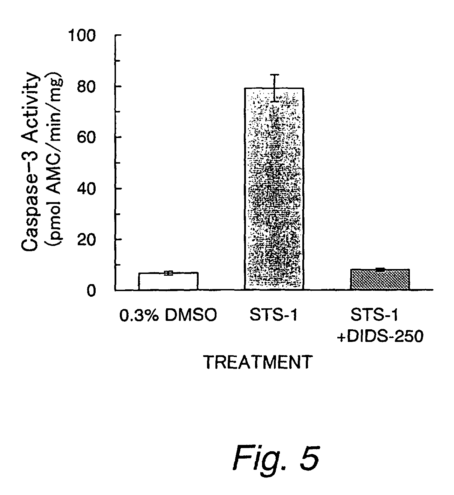

In this Example, tests were conducted as in Example 3 except that 12-well multiwell plates were used, that the test group consisted of a 1 µM staurosporine solution (STS-1) and a mixed solution of the 1 µM staurosporine and 250 µM DIDS (STS-1+DIDS-250), that after 2-hr cultivation following the removal of the conditioned culture medium, cell lysates were prepared using Caspase-3 Assay Kit, Fluorimetric and that in the subsequent stage, the caspase-3 activity of the cells was measured with reference to the formation of specific AMC as an indicator by means of a fluorometric microplate reader (SPECTRA Fluor, Wako Pure Chemical Industries, Ltd., Osaka, Japan) according to the procedure of measurement with the Kit. The result is shown in Fig. 5.

As it turned out, it is elucidated that DIDS suppresses the staurosporine-induced increase in caspase-3 activity.

### Example 5: The protective effect of DIDS on staurosporine-induced cytotoxicity in cultured myocardial cells in HCO₃⁻-free culture medium

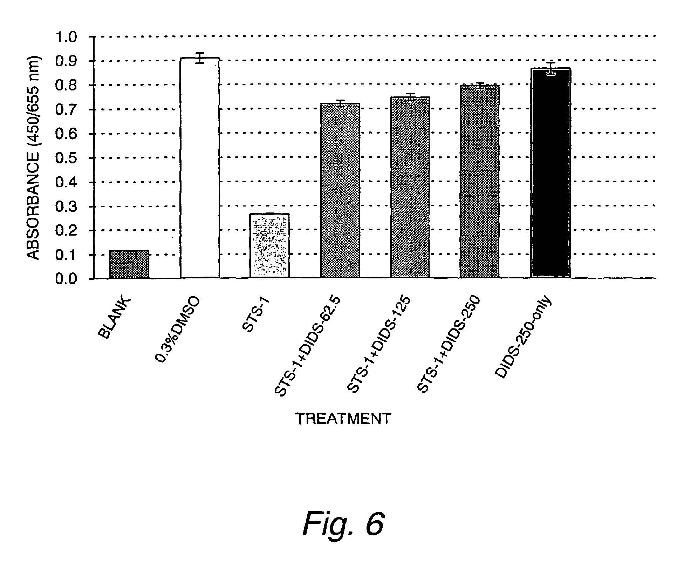

In this Example, tests were conducted as in Example 3 except that an HCO₃⁻-free culture medium was employed as a culture medium based on which the conditioned culture media were prepared and that they were cultivated at 37°C with culture plates being placed in an atmospheric incubator. The result is shown in Fig. 6.

The HCO₃⁻-free culture medium was employed in Example 5 in order to see if the Cl⁻/HCO₃⁻ anion exchangers known as exemplary Cl⁻ channels would participate in the occurrence of apoptosis in cardiovascular cells and in their protection from apoptosis in view of the fact that the function of those exchangers was suppressed by depleting HCO₃⁻.

As a result, the following became clear: even when the HCO₃⁻-free culture medium was substituted for the ordinary culture medium as the base of conditioned culture media, 1) comparable levels of cytotoxicity was induced by staurosporine in the cells to which were added the conditioned culture media containing 1 µM staurosporine; and 2) DIDS suppressed the decrease in cell viability in a dose-dependent manner. From these, the following became clear: even under such conditions that the function of the Cl⁻/HCO₃⁻ anion exchangers as pathways for the permeation of Cl⁻ ions in plasma cell membranes in cultured myocardial cells is considerably decreased, cell death is induced by staurosporine but is suppressed by DIDS in a dose-dependent manner.

### Example 6: The protective effects of SITS on staurosporine-induced cytotoxicity in cultured myocardial cells in HCO₃⁻-free culture medium

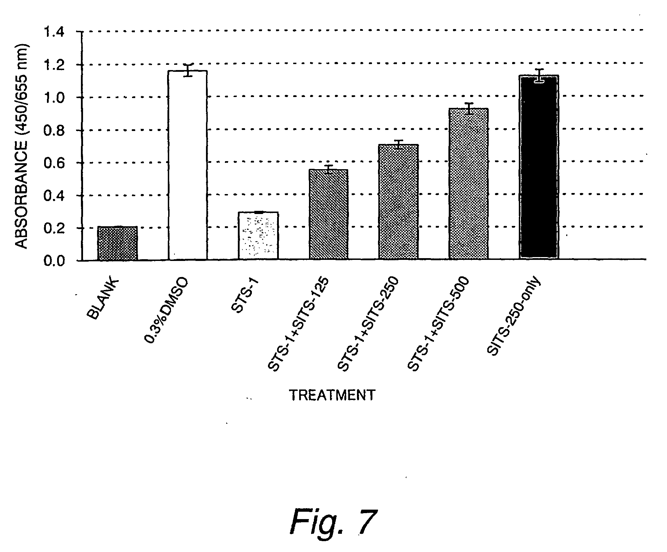

In this Example, tests were conducted as in Example 5 except that the test group consisted of a 1 µM staurosporine solution (STS-1) and a mixed solution of the 1 µM staurosporine and 125, 250 or 500 µM SITS (STS-1+SITS-125, STS-1+SITS-250 or STS-1+SITS-500) and that the control and test groups were cultured on 96-well multiwell plates rather than the 24-well multiwell plates. The result is shown in Fig. 7.

As a result, the following became clear: even when the HCO₃⁻-free culture medium was substituted for the ordinary culture medium as the base of conditioned culture media, 1) comparable levels of cytotoxicity was induced on the 96-well multiwell plates by staurosporine in the cells to which were added the conditioned culture media containing 1 µM staurosporine; and 2) SITS suppressed the decrease in cell viability in a dose-dependent manner. From these, the following became clear: as in Example 5, even under such conditions that the function of the Cl⁻ /HCO₃⁻ anion exchangers as pathways for the permeation of Cl⁻ ions in plasma cell membranes in cultured myocardial cells is considerably decreased on the 96-well multiwell plates, cell death is induced by staurosporine but is suppressed by SITS in a dose-dependent manner.

It therefore became clear that both SITS and DIDS act as Cl⁻ channel blockers and exhibit the apoptosis suppressing effect on myocardial cells.

### Example 7: The protective effect of SITS on staurosporine-induced cytotoxicity of bovine arterial endothelial cell line derived cells

As endothelial cells, a bovine vascular (arterial) endothelium-derived cell line (Accession Number: GM07372) was employed after being cultured in D-MEM supplemented with 10% FBS. A suspension of endothelial cells at an initial density of 5 x 10⁴ cells/mL was dispensed in 24-well multiwell plates in an amount of 0.5 mL per well, cultured for 2 days in 5% CO₂ at 37°C and treated under the following solution conditions.

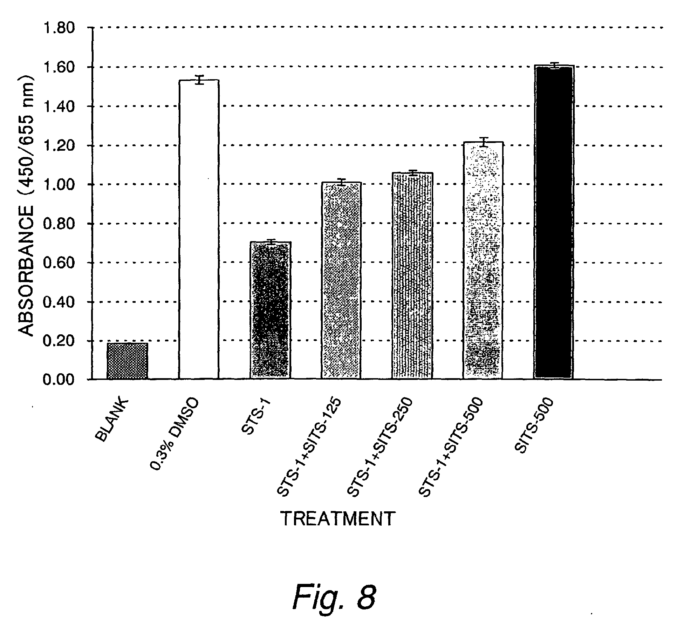

A control group consisted of three culture media, a cell-free medium (blank), a cell-containing medium with 0.3% DMSO that did not contain other substance, and 0.3% DMSO that also contained 500 µM SITS (SITS-500). A test group consisted of two drug-containing culture media, i.e., a solution of 1 µM staurosporine dissolved in 0.3% DMSO (STS-1) and a mixed solution of the 1 µM staurosporine and 125, 250 or 500 µM SITS (STS-1+SITS-125, STS-1+SITS-250, or STS-1+SITS-500). The two groups were cultured in 5% CO₂ at 37°C for 1 hour. Thereafter, the solutions under the respective conditions that had been added to each well were aspirated away and PBS(-) was added in an amount of 0.5 mL per well; after immediately aspirating away the washings, the following treatments were subsequently taken: the wells that had been charged with the staurosporine/SITS mixed solution or the SITS-containing solution condition in the first one hour were given a culture medium (D-MEM supplemented with 10% FBS) containing only the same concentration of SITS and 0.3% DMSO; the wells that had been charged with the other solution conditions in the first one hour were given a culture medium (D-MEM supplemented with 10% FBS) containing only 0.3% DMSO; the respective wells were subsequently cultured for 3 hours in 5% CO₂ at 37°C. Thereafter, the media under the respective conditions were aspirated away and PBS(-) was added in an amount of 0.5 mL per well; after immediately aspirating away the washings, a culture medium (D-MEM supplemented with 10% FBS) was added and cultivation was continued for an additional day in 5% CO₂ at 37°C. Thereafter, cell viability was measured by WST-8. The result is shown in Fig. 8.

As it turned out, even in the case of using the cells derived from the bovine arterial endothelial cell line, it is elucidated that the decrease in cell viability due to staurosporine is suppressed by SITS in a dose-dependent manner.

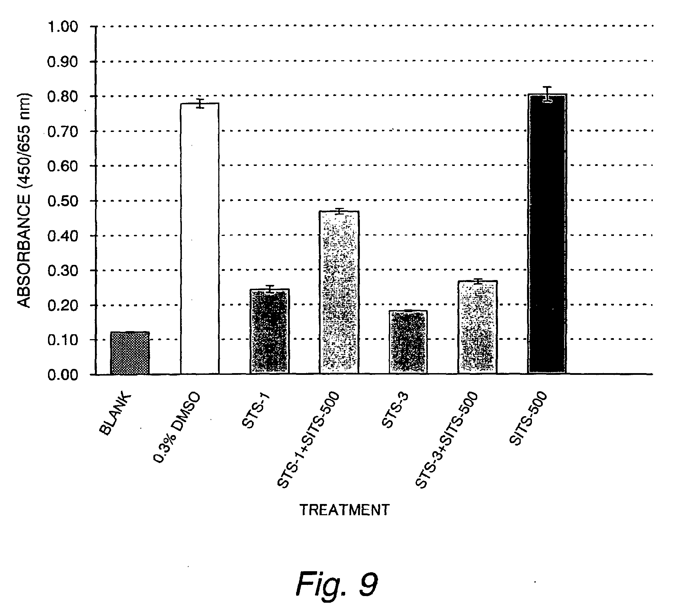

In the next step, tests were conducted at different concentrations of staurosporine in order to see how the dose of staurosporine would influence its cell viability reducing effect and the SITS's suppressive effect on the decrease in cell viability. Specific procedures were as follows. A test group consisted of two drug-containing culture media, i.e., a solution of 1 µM or 3 µM staurosporine dissolved in 0.3% DMSO (STS-1 or STS-3) and a mixed solution of the 1 µM or 3 µM staurosporine and 500 µM SITS (STS-1+SITS-500 or STS-3+SITS-500). Cells were cultured for 3 hours with those solutions; thereafter, the solutions under the respective conditions that had been added to each well were aspirated away and PBS(-) was added in an amount of 0.5 mL per well; after immediately aspirating away the washings, the following treatments were subsequently taken: the wells that had been charged with the staurosporine/SITS mixed solution or the SITS-containing solution condition in the first three hours were given a culture medium (D-MEM supplemented with 10% FBS) containing only the same concentration of SITS and 0.3% DMSO; the wells that had been charged with the other solution conditions in the first three hours were given a culture medium (D-MEM supplemented with 10% FBS) containing only 0.3% DMSO; the respective wells were subsequently cultured for 2 hours in 5% CO₂ at 37°C. All other testing conditions were the same as those employed to obtain the data shown in Fig. 8. The result is shown in Fig. 9.

As it turned out, it is elucidated that the staurosporine-induced decrease in cell viability is effectively suppressed by SITS regardless of staurosporine's concentration.

### INDUSTRIAL APPLICABILITY

It has become clear in the present invention that apoptosis induced in cardiovascular cells can be suppressed by treatment with Cl⁻ channel blockers. As a result, by checking to see if a candidate compound has an apoptosis suppressing effect on apoptogenically stimulated cardiovascular cells, one can determine if said compound can suppress apoptosis in the cardiovascular cells and hence have potential therapeutic or prophylactic effects on cardiac disease.

## Claims

1. A method of screening for therapeutic and/or prophylactic agents of cardiac disease, which comprises the steps of:
inducing apoptosis in cardiovascular cells;
treating the cells with a Cl⁻ channel blocker under test; and
evaluating the therapeutic and/or prophylactic effect of the blocker under test on cardiac disease by checking to see if it can suppress apoptotic cell death in the cardiovascular cells.

2. The method according to claim 1, wherein the cardiovascular cells are cultured myocardial cells and/or cultured vascular endothelial cells.

3. The method according to claim 1 or 2, wherein the ability to suppress apoptotic cell death is assessed by decreased cell viability, decreased cell volume, cytochrome c release, caspase activation, DNA fragmentation, the formation of apoptotic bodies, or the expression of an apoptosis-related specific antigen.

4. The method according to any one of claims 1-3, wherein apoptosis induction and the treatment of the blocker under test are performed simultaneously.

5. The method according to any one of claims 1-3, wherein apoptosis induction and the treatment of the blocker under test are performed consecutively.

6. The method according to any one of claims 1-5, wherein the cardiac disease is ischemic heart disease.

7. A therapeutic agent of cardiac disease comprising a Cl⁻ channel blocker as an active ingredient.
